# EUROPEAN PATENT APPLICATION

(11) **EP 3 653 159 A1**
(43) Date of publication of application: **20.05.2020**
(21) Application number: 18832741.5
(22) Date of filing: 09.07.2018
(51) Int. Cl.: A61B 34/30

(54) **SURGICAL INSTRUMENT HAVING ELECTRICALLY-DRIVEN CLAMPING MECHANISM**

(30) Priority: 13.07.2017 CN 201710570845
(71) Applicant: Shanghai Yisi Medical Technology Co., Ltd., Shanghai 201318 (CN); Yisi Suzhou Medical Technology Co., Ltd., Suzhou City, Jiangsu 215163 (CN)
(72) Inventor: LI, Zhidong, Shanghai 201318 (CN); ZHANG, Yu, Shanghai 201318 (CN); CHANG, Wangtao, Shanghai 201318 (CN)
(74) Representative: Strachan, Victoria Jane
(86) International application number: PCT/CN2018/094988
(87) International publication number: WO 2019/011213

(57) **Abstract**

The present application provides a surgical instrument with an electric drive clamping mechanism, comprising a clamping arm and an electric drive clamping mechanism, wherein the electric drive clamping mechanism comprises a moving component, a static component and a driving component; the surgical instrument is characterized in that: the driving component comprises a magnetizing body and a magnetized body; one of the magnetizing body and the magnetized body is connected with the moving component, and the other one is connected with the static component; the moving component is connected with the clamping arm; the magnetizing body generates a magnetic force when current is connected, the direction and degree of the magnetic force can be changed by changing current direction and degree; the magnetized body is attracted or repelled by the magnetic force generated by the magnetizing body, thereby driving the moving component to get close to or keep far away from the static component and further driving the clamping arm to close or open. The surgical instrument with the electric driving clamping mechanism and a surgical system can dynamically adjust the clamping force upon the using condition so as to reach better cutting and coagulating effects; and compared with the prior art, the surgical instrument has the advantages of simple structure and low cost, can be applied to a robot operation as a terminal executor, and is convenient in operation, and good for large-scale popularization and promotion.

## Description

### TECHNICAL FIELD

The present application relates to a surgical instrument, and particularly relates to a surgical instrument with an electric drive clamping mechanism and a surgical system including the surgical instrument.

### BACKGROUD

Currently, clinical application of active surgical instruments has already been very popular. Common active surgical instruments include high frequency electrotomes, ultrasonic scalpels, laser scalpels and plasma surgical instruments. Application of high frequency electrotomes and ultrasonic scalpels is the widest, for such kind of instruments, heat effect is mainly utilized to cut tissues (an ultrasonic scalpel also has a mechanical cutting effect) and coagulate and stop bleeding. It is known that cells of a human body will die when temperature is higher than 60°C, protein will denature when temperature is between 60°C and 90°C; while water molecules in cells will evaporate when temperature is higher than 100°C, causing cracking of cell walls; and human tissues will be carbonized due to complete dehydration when temperature reaches 200°C. Active surgical instruments such as high frequency electrotomes and ultrasonic scalpels utilize a heat effect to vaporize water molecules in tissues, denature protein, crack cells and carbonize tissues, so that the tissues are cut open or coagulated, and then blood vessel occlusion is realized. For such kind of active surgical instrument, main factors affecting cutting and coagulating effects include temperature applied to the tissues, clamping force to the tissues and acting time.

Clamping mechanisms of conventional active surgical instruments are manually driven by a doctor or an operator, for example, manually driven clamping mechanisms in CN104224279A (Reference 1), CN101396302A (Reference 2) and CN101495050A (Reference 3). Such manually driven clamping mechanisms are generally formed by components such as a trigger or a handheld arm, a cam structure or a link mechanism and a spring. When a doctor or an operator grasps a trigger or a handheld arm, movement of the trigger can be transmitted to a clamping arm at a terminal by the conversion of a cam or a linkage and a spring mechanism, so that the clamping arm can close and clamp a tissue to be cut. This type of manually driven clamping mechanism is relatively reliable and effective in conventional surgery, and brings great convenience for the manual operation of a doctor and an operator.

However, along with the development of technology, conventional manually driven clamping mechanisms have gradually presented deficiencies. For example, some research institutions at home and abroad have already developed a surgical robot to a practical stage, and then active surgical instruments using manually driven clamping mechanisms cannot be used as a terminal executing instrument in surgery operated by a robot. Moreover, as for active surgical instruments, there is an ongoing desire for higher cutting speed and better blood vessel coagulation effect, wherein clamping force is a key factor affecting cutting speed and coagulating effect. Conventional manually driven clamping mechanisms can only generate relatively fixed clamping force, and cannot dynamically adjust the clamping force according to use conditions to achieve a better cutting and coagulating effect. Although some highly skilled doctors can manually adjust a clamping force in a cutting or coagulating process to achieve a better use effect, there is high requirement on the personal skills and experience of doctors, thereby making it difficult to popularize in a large scale.

Therefore, active surgical instruments with a clamping mechanism adjusted by electric control has become a research direction of technicians. CN104582617A (Reference 4) and CN104582627A (Reference 5) disclose a plurality of active surgical instruments with electric drive clamping mechanisms, however, these instruments all need a motor driven clamping mechanism, and are complicated in structure and high in cost.

### SUMMARY

In order to solve the foregoing technical problems, the present application provides a surgical instrument with an electric drive clamping mechanism and a corresponding surgical system, which have the advantages of being simple in structure and low in cost in comparison with the prior art.

According to an aspect of the present application, a surgical instrument with an electric drive clamping mechanism is provided, including a clamping arm and an electric drive clamping mechanism, wherein the electric drive clamping mechanism includes a moving component, a static component and a driving component; the surgical instrument is characterized in that: the driving component includes a magnetizing body and a magnetized body; one of the magnetizing body and the magnetized body is connected with the moving component, and the other one is connected with the static component; the moving component is connected with the clamping arm; the magnetizing body generates a magnetic force when current is connected, the direction and degree of the magnetic force can be changed by changing current direction and degree, and the magnetized body is attracted or repelled by the magnetic force generated by the magnetizing body, thereby driving the moving component to get close to or keep far away from the static component and further driving the clamping arm to close or open.

In an implementation mode, the magnetizing body is connected with the static component, and the magnetized body is connected with the moving component.

In another implementation mode, the magnetizing body is connected with the moving component, and the magnetized body is connected with the static component.

Further, the driving component may optionally include a driving elastomer; one end of the driving elastomer being connected to the static component, the other end being connected to the moving component, and the driving elastomer having elasticity.

Further, the driving component may also include a limiting elastomer; the limiting elastomer having elasticity, and being mounted on the moving component.

In an implementation mode, the limiting elastomer can obstruct an opposite movement between the magnetized body and the magnetizing body.

In another implementation mode, the limiting elastomer can obstruct an antitropic movement between the magnetized body and the magnetizing body.

Specifically, the foregoing surgical instrument may be an ultrasonic surgical instrument, a high frequency electrotome, a plasma surgical instrument or a laser scalpel. Further, the surgical instrument may be an ultrasonic surgical instrument, the ultrasonic surgical instrument may also include a scalpel bar, the scalpel bar having a scalpel head at a far end, the clamping arm being respectively connected with the far end of the moving component and the far end of the static component by two rotary kinematic pairs, and when the moving component moves towards the near end or the far end relative to the static component, the clamping arm being driven to close or open relative to the scalpel head, so as to clamp a tissue and apply a certain clamping force to the clamped tissue.

Further, the driving component may receive a control current signal of a clamping mechanism to control the moving component to move towards a near end or a far end relative to the static component.

Further, the moving component may include an inner tube, a connecting seat and a fastening nut; the inner tube being located outside the scalpel bar and extending along the longitudinal direction of the scalpel bar, the connecting seat sleeves on the inner end of the inner tube and being fixedly connected with the inner tube, and the fastening nut being fixedly connected to the outer periphery of the connecting seat.

In an implementation mode, a groove is formed in the outer side of the near end of the inner tube, and a bulge is formed at a corresponding position of the inner side of the near end of the connecting seat, the bulge being buckled in the groove to realize buckled fixed connection of the inner tube with the connecting seat.

In an implementation mode, the fastening nut includes an internal thread, an external thread is formed at a corresponding position of the connecting seat, and the internal thread and the external thread are mutually connected to fasten the fastening nut with the connecting seat.

Further, the static component may include an outer tube, a base, a shell and a hinge pin; the outer tube being located outside the inner tube and extending along the longitudinal direction of the inner tube; the base including a hole and sleeves on the near end of the outer tube, the base being fixedly connected with the shell in the direction of a longitudinal axis; holes vertical to the longitudinal axis being formed in corresponding positions on the base, the outer tube, the inner tube and on the scalpel bar; after the hinge pin penetrates through these holes in sequence, the widths of holes in the base, the outer tube and the scalpel bar in the direction of the longitudinal axis of the scalpel bar are equal to or slightly greater than the width of the hinge pin in the direction of the longitudinal axis, while the width of the hole in the inner tube in the direction of the longitudinal axis is greater than the width of the hinge pin in the direction of the longitudinal axis. According to the foregoing structure, the movement of the base, the outer tube and the scalpel towards the near end or the far end relative to the shell is limited, while the inner tube can move towards the near end or the far end relative to the shell.

Further, the hinge pin may be cylindrical, holes in the base, the outer tube and the scalpel bar may be round holes, diameters of the round holes being equal to or slightly greater than the diameter of the hinge pin, while a hole in the inner tube is a waist-shaped hole, the length of the hole along the direction of the longitudinal axis is greater than the diameter of the hinge pin, while the width of the hole vertical to the direction of the longitudinal axis is equal to or slightly greater than the diameter of the hinge pin.

Further, a groove may be provided at the near end of the base, a boss being provided at a corresponding position of the far end of the shell, and the boss of the shell being stuck into the groove in the base to realize fixed connection of the base and the shell in the direction of the longitudinal axis.

Further, the magnetizing body may be mounted on the shell, one end of the driving elastomer being in contact with the magnetizing body, and the other end being in contact with the fastening nut and stops opposite movement between the fastening nut and the magnetizing body depending on self elasticity.

In an implementation mode, the driving component includes a limiting elastomer, the limiting elastomer has elasticity, and the limiting elastomer and the magnetized body are mounted on the moving component.

Further, the magnetized body and the limiting elastomer may be mounted between the connecting seat and the fastening nut, so that the moving component can be driven to move towards a near end or a far end when the magnetized body moves towards a near end or a far end.

In another implementation mode, the driving elastomer stops an antitropic movement between the static component and the moving component.

In an implementation mode, the limiting elastomer stops an opposite movement between the magnetized body and the magnetizing body.

In another implementation mode, the limiting elastomer stops an antitropic movement between the magnetized body and the magnetizing body.

Specifically, the magnetizing body may be formed by winding a conductor coil on a silicon steel sheet.

Specifically, the magnetized body may be formed by pure iron, soft magnetic ferrite or low-carbon steel.

In an implementation mode, the magnetized body is a permanent magnet.

In another implementation mode, the magnetized body is an electromagnet, and can generate magnetic force when current is connected, and the direction and degree of the magnetic force can be changed by changing current direction and degree.

In an implementation mode, the driving elastomer and the limiting elastomer can be made to be a wave spring or a cylindrical spring by using a material with low magnetoconductivity.

In another implementation mode, the driving elastomer and the limiting elastomer are made from an elastic colloid material.

According to a second aspect of the present application, a surgical system with an electric drive clamping mechanism is provided, including the foregoing surgical instrument and a generator, the generator generating a clamping mechanism control current and transmitting to the electric drive clamping mechanism.

Further, the surgical system may be a robot surgical system, current direction and degree in the magnetizing body and/or the magnetized body being automatically adjusted by the robot surgical system.

The surgical instrument with an electric drive clamping mechanism and the surgical system according to the present application can dynamically adjust a clamping force according to use conditions to achieve a better cutting and coagulating effect, and have the advantages of being simple in structure and low in cost in comparison with published implementation schemes in reference patents and other existing technologies. Moreover, the surgical instrument with an electric drive clamping mechanism according to the present application can be applied to robot surgery as a terminal executing instrument, and is convenient in operation and favorable for large-scale popularization.

### BRIEF DESCRIPTION OF THE DRAWINGS

FIG. 1 is a structure diagram of an ultrasonic surgical system according to an implementation mode of the present application;
FIG. 2 is a specific schematic structure diagram of an ultrasonic surgical instrument with an electric drive clamping mechanism included in the ultrasonic surgical system in FIG. 1; and
FIG. 3 is a specific schematic structure diagram of an electric drive clamping mechanism in FIG. 2.

### DESCRIPTION OF THE EMBODIMENTS

The following clearly and completely describes the technical schemes of embodiments of the present invention. However, the described embodiments are merely some of the embodiments of the present invention rather than all of the embodiments. All other embodiments obtained by a person of ordinary skill in the art based on the embodiments of the present invention without creative efforts shall fall within the protection scope of the present disclosure.

For the avoidance of doubt, although the present application describes an electric drive clamping structure of the present application in details by taking an ultrasonic scalpel as an embodiment, the present application is applicable to all active surgical instruments needing a clamping operation to complete cutting and coagulating on tissues of the human body. Because a clamping force of a jaw not only is an important indicator affecting performances for ultrasonic surgical instruments, but also is a very important indicator for other active surgical instruments including high frequency electrotomes, plasma surgical instruments or a laser scalpels, dynamic adjustment of the clamping force according to the tissue cutting and coagulation conditions in surgery is beneficial for such kind of instruments, and suck kind of instruments all have the requirement of being applied to surgery of a robot.

For the convenience of description, "near end" in the whole application refers to an end close to an operator after the operator holds an instrument, and "far end" refers to an end far away from the operator after the operator holds the instrument.

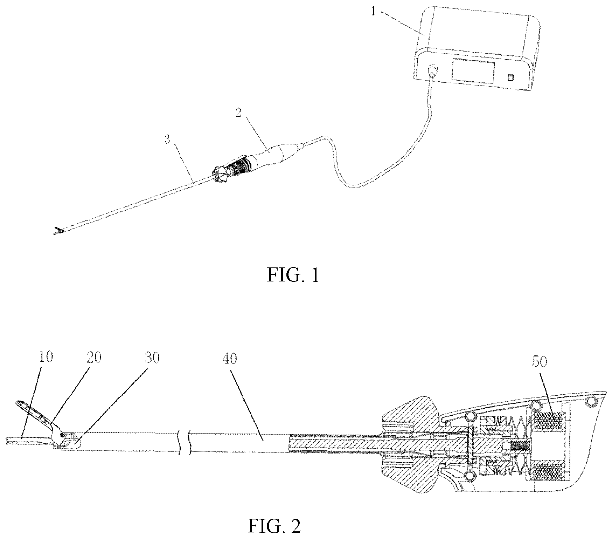

Refer to FIG. 1, an ultrasonic surgical system according to an implementation mode of the present application is shown, including a generator 1, a transducer 2 and an ultrasonic surgical instrument 3, wherein the ultrasonic surgical instrument 3 includes an electric drive clamping mechanism. The generator 1 generates ultrasonic control current and clamping mechanism control current and transmits to the transducer 2, and the transducer 2 converts the received ultrasonic control current into mechanism vibration and transmits to the jaw or scalpel head of the ultrasonic surgical instrument 3 on one hand, and transmits the clamping mechanism control current to an electric drive clamping mechanism in the ultrasonic surgical instrument 3 on the other hand. The ultrasonic surgical instrument 3 receives the ultrasonic vibration current and the clamping mechanism control current, and clamps a tissue of a human body and performs ultrasonic cutting and coagulating on the clamped tissue via a jaw at a far end.

Refer to FIG. 2, an ultrasonic surgical instrument 3 with an electric drive clamping mechanism shown in FIG. 1 is shown in details, including a scalpel bar 10, a clamping arm 20, a moving component 30, a static component 40 and a driving component 50. The scalpel bar 10 is fixedly connected with the transducer 2 at a near end (an end close to an operator), and is connected by thread in an implementation mode shown in FIG. 2, the scalpel bar 10 receives mechanical vibration generated by the transducer 2 to generate ultrasonic resonance, and the scalpel bar 10 has a scalpel head applying ultrasonic energy to a clamped tissue at a far end (an end far away from an operator). The clamping arm 20 can close or open relative to the scalpel head to clamp a tissue and apply a certain clamping force to the clamped tissue.

The driving component 50 receives a clamping mechanism control current signal transmitted by the transducer 2 to control the moving component 30 to move towards a near end or a far end relative to the static component 40. The clamping arm 20 is respectively connected with the far end of the moving component 30 and the far end of the static component 40 by two rotary kinematic pairs, and when the moving component 30 moves towards the near end or the far end relative to the static component 40, the clamping arm 20 is driven to close or open relative to a scalpel head of the scalpel bar 10, so as to clamp a tissue and apply a certain clamping force to the clamped tissue.

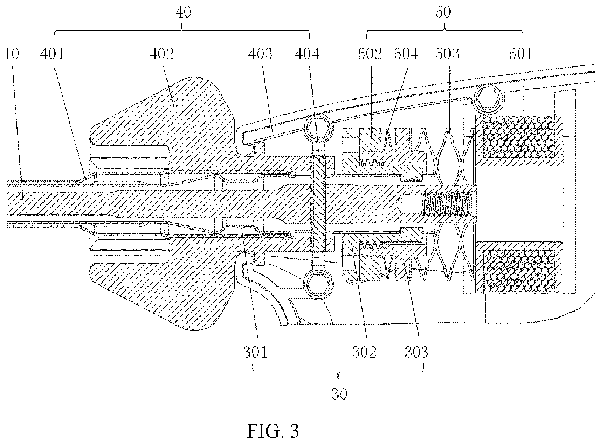

Refer to FIG. 3, a specific schematic structure diagram of an electric drive clamping mechanism in FIG. 2 is shown in details, and the electric drive clamping mechanism includes a moving component 30, a static component 40 and a driving component 50. The moving component 30 includes an inner tube 301, a connecting seat 302 and a fastening nut 303; the inner tube 301 is located outside a scalpel bar 10 and extends along the longitudinal direction of the scalpel bar 10, the connecting seat 302 sleeves on the near end of the inner tube 301 and is buckled in the inner tube 301 by a buckle, referring to FIG. 3, a groove is formed in the outer side of the near end of the inner tube 301, a bulge is formed at a corresponding position of the inner side of the near end of the connecting seat 302, and the bulge is buckled in the groove to fixedly connect the inner tube 301 with the connecting seat 302. The fastening nut 303 is fixedly connected to the outer periphery of the connecting seat 302, and in an implementation mode shown in FIG. 2, the fastening nut 303 includes an internal thread, an external thread is formed at a corresponding position of the connecting seat 302, and the internal thread and the external thread are mutually connected to fasten the fastening nut 303 with the connecting seat 302.

Continue to refer to FIG. 3, the static component 40 includes an outer tube 401, a base 402, a shell 403 and a hinge pin 404; the outer tube 401 is located outside the inner tube 301 and extends along the longitudinal direction of the inner tube 301; the base 402 includes a hole and sleeves on the near end of the outer tube 401, a groove is formed in the near end of the base 402, a boss is provided on a corresponding position of the far end of the shell 403, and the boss of the shell 403 is stuck into the groove in the base 402 to realize fixed connection of the base 402 with the shell 403 along the direction of a longitudinal axis. Holes vertical to the longitudinal axis are formed in the base 402, the outer tube 401, the inner tube 301 and the scalpel bar 10, and the hinge pin 404 penetrates through these holes in sequence. Holes in the base 402, the outer tube 401 and the scalpel bar 10 are all round holes, the diameters of the round holes are equal to or slightly greater than the diameter of the hinged pin 404, while a hole in the inner tube 301 is a waist-shaped hole, the width of the hole along the direction of the longitudinal axis of the inner tube 301 is greater than the diameter of the hinge pin, while the width of the hole vertical to the direction of the longitudinal axis is equal to or slightly greater than the diameter of the hinge pin. With such a structure, the movement of the base 402, the outer tube 401 and the scalpel bar 10 towards the near end or the far end relative to the shell 403 is limited, while the inner tube 301 can move towards the near end or the far end relative to the shell.

Continue to refer to FIG. 3, the driving component 50 includes a magnetizing body 501, a magnetized body 502, a driving elastomer 503 and a limiting elastomer 504. The magnetizing body 501 is mounted on the shell 403, and in an implementation mode shown in FIG. 3, the magnetizing body 501 is located at the near end of the fastening nut 303. One end of the driving elastomer 503 is in contact with the magnetizing body 501, and the other end is in contact with the fastening nut 303 and stops opposite movement between the fastening nut 303 and the magnetizing body 501 depending on self elasticity. The magnetized body 502 and the limiting elastomer 504 are mounted on the moving component 30, for example, as shown in FIG. 3, being mounted between the connecting seat 302 and the fastening nut 303, so that the moving component 30 is driven to move towards a near end or a far end when the magnetized body 502 moves towards a near end or a far end.

A working process of an electric drive clamping mechanism is as follows: when the magnetizing body 501 receives a clamping mechanism control current signal transmitted by the transducer 2, that is when being electrified, the magnetizing body 501 generates a magnetic force to attract the magnetized body 502 to move towards a near end, so as to drive the moving component 30 to move towards the near end, and the moving component 30 drives the clamping arm 20 to gradually close relative to the scalpel head of the scalpel bar 10 by rotary kinematic pairs. The degree of a magnetic force generated by the magnetizing body 501 is controlled by the degree of electrifying current in the magnetizing body 501, so as to correspondingly control the distance of the moving component 30 moving towards the near end, and further control the closing degree of the clamping arm 20 relative to the scalpel head of the scalpel bar 10 and the degree of a clamping force between the two. Specifically, when electrifying current in the magnetizing body 501 is gradually increased, the driving elastomer 503 is gradually compressed, closing of the clamping arm 20 relative to the scalpel bar 10 is tighter, and a clamping force between the two is gradually strengthened, until the inner tube 301 is stopped by the hinged pin 404 to be limited in further moving towards the near end, at the moment, the driving elastomer 503 is compressed to a maximal value, and a clamping force between the clamping arm 20 and the scalpel head of the scalpel bar 10 also reaches a maximal value; at the moment, if current in the magnetizing body 501 is further increased, the magnetized body 502 will compress the limiting elastomer 504, and the compressing force of the limiting elastomer 504 limits the magnetized body in further moving towards the near end. When electrifying current in the magnetizing body 501 is gradually reduced, the driving elastomer 503 is elongated due to self elastic effect, so as to push the moving component 30 to move towards a far end, and drive the clamping arm 20 to gradually open relative to the scalpel head of the scalpel bar 10, and a clamping force between the two is gradually weakened, until being finally opened and separated. With the foregoing electric drive clamping mechanism, control on closing and opening actions between the clamping arm 20 and the scalpel head of the scalpel bar 10 via current degree can be realized, and the degree of a clamping force in clamping can be controlled.

The magnetizing body 501 is formed in a structure form of generating magnetic force by current, and in an implementation mode, the magnetizing body 501 is formed by winding a conductor coil on a silicon steel sheet. The magnetized body 502 is formed by materials with high magnetoconductivity, such as pure iron, soft magnetic ferrite or low-carbon steel, or is a permanent magnet, and may be also an electromagnet, and degree of magnetic force of the magnetized body is controlled by current. The driving elastomer 503 and the limiting elastomer 504 can be made to be a wave spring or a cylindrical spring by using a material with low magnetoconductivity. Besides, all other components need to be made from materials with low magnetoconductivity or non-magnetic-conductive materials.

The surgical instrument with an electric driving clamping mechanism and a surgical system according to the present application can dynamically adjust the clamping force upon the using condition so as to reach better cutting and coagulating effects, and have the advantages of being simple in structure and low in cost in comparison with published implementation schemes in reference patents and other existing technologies. Moreover, the surgical instrument with an electric drive clamping mechanism according to the present application can be applied to robot surgery as a terminal executing instrument, and is convenient in operation and favorable for large-scale popularization.

It should to be noted that implementation schemes in the accompanying drawings are merely representative embodiments of the present application, a person skilled in the art may easily understand that the protection scope of the present application is not merely limited in a scope defined by implementation modes in the accompanying drawings, and combination, transformation and variation for implementation modes in the drawings all fall within the protection scope of the present application.

The foregoing disclosed are merely several preferred embodiments of the present application, of course, the protection scope of the present application should be not limited hereby, therefore, equivalent variations made according to claims of the present application still belong to a coverage scope of the present application.

## Claims

1. A surgical instrument with an electric drive clamping mechanism, comprising a clamping arm and an electric drive clamping mechanism, wherein the electric drive clamping mechanism comprises a moving component, a static component and a driving component; the surgical instrument is **characterized in that**: the driving component comprises a magnetizing body and a magnetized body; one of the magnetizing body and the magnetized body is connected with the moving component, and the other one is connected with the static component; the moving component is connected with the clamping arm; the magnetizing body generates a magnetic force when current is connected, the direction and degree of the magnetic force can be changed by changing current direction and degree; the magnetized body is attracted or repelled by the magnetic force generated by the magnetizing body, thereby driving the moving component to get close to or keep far away from the static component and further driving the clamping arm to close or open.

2. The surgical instrument with an electric drive clamping mechanism according to claim 1, wherein the driving components receives a control current signal of a clamping mechanism to control the moving component to move towards a near end or a far end relative to the static component.

3. The surgical instrument with an electric drive clamping mechanism according to claim 2, wherein the magnetizing body is connected with the static component, and the magnetized body is connected with the moving component.

4. The surgical instrument with an electric drive clamping mechanism according to claim 2, wherein the magnetizing body is connected with the moving component, and the magnetized body is connected with the static component.

5. The surgical instrument with an electric drive clamping mechanism according to claim 2, wherein the driving component also comprises a driving elastomer; one end of the driving elastomer being connected to the static component, the other end being connected to the moving component, and the driving elastomer having elasticity.

6. The surgical instrument with an electric drive clamping mechanism according to claim 5, wherein the driving elastomer stops an antitropic movement between the static component and the moving component.

7. The surgical instrument with an electric drive clamping mechanism according to claim 5, wherein the driving elastomer stops an opposite movement between the static component and the moving component.

8. The surgical instrument with an electric drive clamping mechanism according to claim 5, wherein the driving component also comprises a limiting elastomer, the limiting elastomer having elasticity, and the limiting elastomer being mounted on the moving component.

9. The surgical instrument with an electric drive clamping mechanism according to claim 8, wherein the limiting elastomer can obstruct an opposite movement between the magnetized body and the magnetizing body.

10. The surgical instrument with an electric drive clamping mechanism according to claim 8, wherein the limiting elastomer can obstruct an antitropic movement between the magnetized body and the magnetizing body.

11. The surgical instrument with an electric drive clamping mechanism according to claim 5, wherein the surgical instrument is an ultrasonic surgical instrument, comprising a scalpel bar, the scalpel bar having a scalpel head at a far end, the clamping arm being respectively connected with the far end of the moving component and the far end of the static component by two rotary kinematic pairs, and when the moving component moves towards the near end or the far end relative to the static component, the clamping arm being driven to close or open relative to the scalpel head, so as to clamp a tissue and apply a certain clamping force to the clamped tissue.

12. The surgical instrument with an electric drive clamping mechanism according to claim 11, wherein the moving component comprises an inner tube, a connecting seat and a fastening nut; the inner tube being located outside the scalpel bar and extending along the longitudinal direction of the scalpel bar, the connecting seat sleeving on the inner end of the inner tube and being fixedly connected with the inner tube, and the fastening nut being fixedly connected to the outer periphery of the connecting seat.

13. The surgical instrument with an electric drive clamping mechanism according to claim 12, wherein a groove is formed in the outer side of the near end of the inner tube, and a bulge is formed at a corresponding position of the inner side of the near end of the connecting seat, the bulge being buckled in the groove to realize buckled fixed connection of the inner tube with the connecting seat.

14. The surgical instrument with an electric drive clamping mechanism according to claim 12, wherein the fastening nut comprises an internal thread, an external thread is formed at a corresponding position of the connecting seat, and the internal thread and the external thread are mutually connected to fasten the fastening nut with the connecting seat.

15. The surgical instrument with an electric drive clamping mechanism according to claim 12, wherein the static component comprises an outer tube, a base, a shell and a hinge pin; the outer tube being located outside the inner tube and extending along the longitudinal direction of the inner tube; the base comprising a hole and sleeving on the near end of the outer tube, the base being fixedly connected with the shell in the direction of a longitudinal axis; holes vertical to the longitudinal axis being formed in corresponding positions on the base, the outer tube, the inner tube and on the scalpel bar; after the hinge pin penetrates through these holes in sequence, the widths of holes in the base, the outer tube and the scalpel bar in the direction of the longitudinal axis of the scalpel bar being equal to or slightly greater than the width of the hinge pin in the direction of the longitudinal axis, while the width of the hole in the inner tube in the direction of the longitudinal axis being greater than the width of the hinge pin in the direction of the longitudinal axis, so that the movement of the base, the outer tube and the scalpel bar towards the near end or the far end relative to the shell is limited, while the inner tube can move towards the near end or the far end relative to the shell.

16. The surgical instrument with an electric drive clamping mechanism according to claim 15, wherein the hinge pin is cylindrical, and holes in the base, the outer tube and the scalpel bar are round holes, diameters of the round holes being equal to or slightly greater than the diameter of the hinge pin, while a hole in the inner tube is a waist-shaped hole, the length of the hole along the direction of the longitudinal axis being greater than the diameter of the hinge pin, while the width of the hole vertical to the direction of the longitudinal axis being equal to or slightly greater than the diameter of the hinge pin.

17. The surgical instrument with an electric drive clamping mechanism according to claim 15, wherein a groove is provided at the near end of the base, a boss is provided at a corresponding position of the far end of the shell, and the boss of the shell is stuck into the groove in the base to realize fixed connection of the base and the shell in the direction of the longitudinal axis.

18. The surgical instrument with an electric drive clamping mechanism according to claim 15, wherein the magnetizing body is mounted on the shell, one end of the driving elastomer is in contact with the magnetizing body, the other end is in contact with the fastening nut and stops opposite movement between the fastening nut and the magnetizing body depending on self elasticity.

19. The surgical instrument with an electric drive clamping mechanism according to claim 15, wherein the driving component also comprises a limiting elastomer, the limiting elastomer having elasticity.

20. The surgical instrument with an electric drive clamping mechanism according to claim 19, wherein the magnetized body and the limiting elastomer are mounted on the moving component, and the limiting elastomer stops an opposite movement between the magnetized body and the magnetizing body.

21. The surgical instrument with an electric drive clamping mechanism according to claim 20, wherein the magnetized body and the limiting elastomer are mounted between the connecting seat and the fastening nut.

22. The surgical instrument with an electric drive clamping mechanism according to claim 19, wherein the limiting elastomer stops an antitropic movement between the magnetized body and the magnetizing body.

23. The surgical instrument with an electric drive clamping mechanism according to any one of claims 1-22, wherein the surgical instrument is a high frequency electrotome, a plasma surgical instrument or a laser scalpel.

24. The surgical instrument with an electric drive clamping mechanism according to any one of claims 1-22, wherein the magnetizing body is formed by winding a conductor coil on a silicon steel sheet.

25. The surgical instrument with an electric drive clamping mechanism according to any one of claims 1-22, wherein the magnetized body is formed by pure iron, soft magnetic ferrite or low-carbon steel.

26. The surgical instrument with an electric drive clamping mechanism according to any one of claims 1-22, wherein the magnetized body is a permanent magnet.

27. The surgical instrument with an electric drive clamping mechanism according to any one of claims 1-22, wherein the magnetized body is an electromagnet, and can generate magnetic force when current is connected, and the direction and degree of the magnetic force can be changed by changing current direction and degree.

28. The surgical instrument with an electric drive clamping mechanism according to any one of claims 1-22, wherein the driving elastomer and the limiting elastomer can be made to be a wave spring or a cylindrical spring by using a material with low magnetoconductivity.

29. The surgical instrument with an electric drive clamping mechanism according to any one of claims 1-22, wherein the driving elastomer and the limiting elastomer are made from an elastic colloid material.

30. A surgical system with an electric drive clamping mechanism, comprising the surgical instrument according to any one of claims 1-29 and a generator, the generator generating a clamping mechanism control current and transmitting to the electric drive clamping mechanism.

31. The surgical system with an electric drive clamping mechanism according to claim 30, wherein the surgical system is a robot surgical system, current direction and degree in the magnetizing body and/or the magnetized body being automatically adjusted by the robot surgical system.
